# EUROPEAN PATENT SPECIFICATION

(11) **EP 4 017 434 B1**
(45) Date of publication and mention of the grant of the patent: **14.06.2023**
(21) Application number: 20765404.7
(22) Date of filing: 19.08.2020
(51) Int. Cl.: A61F 5/453, A61F 5/451, A61F 5/44, A61B 10/00

(54) **A FLUID COLLECTION ASSEMBLY INCLUDING A REUSABLE FLUID IMPERMEABLE BARRIER AND AT LEAST ONE SINGLE USE WICKING CARTRIDGE**
FLÜSSIGKEITSSAMMELANORDNUNG MIT EINER WIEDERVERWENDBAREN FLÜSSIGKEITSUNDURCHLÄSSIGEN BARRIERE UND MINDESTENS EINER EINWEG-KASSETTE MIT DOCHTWIRKUNG
ENSEMBLE DE COLLECTE DE FLUIDE COMPRENANT UNE BARRIÈRE IMPERMÉABLE AUX FLUIDES RÉUTILISABLE ET AU MOINS UNE CARTOUCHE À EFFET DE MÈCHE À USAGE UNIQUE

(30) Priority: 20.08.2019 US 201962889149 P
(43) Date of publication of application: 29.06.2022
(73) Proprietor: Purewick Corporation, El Cajon, California 92020 (US)
(72) Inventor: EVANS, Megan, Grove City, Ohio 43123 (US); TRULLENQUE, Hollie, Houston, Texas 77027 (US); ROOT, Michele, Malvern, Pennsylvania 19355 (US); JOHANNES, Ashley, Atlanta, Georgia 30252 (US)
(74) Representative: Hoffmann Eitle
(86) International application number: PCT/US2020/046914
(87) International publication number: WO 2021/034886

(56) References cited:
- WO-A1-2016/051385
- US-A1- 2019 142 624
- US-B1- 6 764 477
- US-B1- 9 308 118

## Description

### BACKGROUND

A person or animal may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, urinary catheters can be uncomfortable, painful, and can lead to complications, such as infections. Additionally, bed pans, which are receptacles used for the toileting of bedridden patients are sometimes used. However, bedpans can be prone to discomfort, spills, and other hygiene issues. US2019/0142624A1 discloses a fluid collection assembly with a fluid (urine) impermeable barrier defining a chamber that contains wicking material introduced into the chamber through an elongate opening in the barrier and retained in the chamber with tapes at the ends of the opening.

### SUMMARY

The invention is defined in claims 1 and 13 below. The dependent claims are directed to optional features and preferred embodiments. In an embodiment, a fluid collection assembly is disclosed. The fluid collection assembly includes a reusable fluid impermeable barrier including a first end and a second end opposite the first end. The reusable fluid impermeable barrier includes at least one inner surface defining a chamber extending between the first end and the second end. The reusable fluid impermeable barrier also defines an outlet and at least one opening spaced from the outlet that both provide access to the chamber. The fluid collection assembly also includes at least one single use wicking cartridge configured to receive fluid. The reusable fluid impermeable barrier is configured to be transformed between a first configuration and a second configuration. The reusable fluid impermeable barrier does not allow the at least one single use wicking cartridge to be inserted and/or removed from the chamber when the reusable fluid impermeable barrier is in the first configuration and the reusable fluid impermeable barrier allows the at least one single use wicking cartridge to be inserted and/or removed from the chamber when the reusable fluid impermeable barrier is in the second configuration.

In an embodiment, a method of using a fluid collection assembly is disclosed. The method includes providing the fluid collection assembly including a reusable fluid impermeable barrier and a first single use wicking cartridge. The reusable fluid impermeable barrier includes a first end and a second end opposite the first end. The reusable fluid impermeable barrier including at least one inner surface defines a chamber extending between the first end and the second end. The reusable fluid impermeable barrier also defines an outlet and at least one opening spaced from the outlet that both provide access to the chamber. The reusable fluid impermeable barrier is configured to be transformed between a first configuration and a second configuration. The first single use wicking cartridge is configured to receive fluid. The method also includes, when the reusable fluid impermeable barrier is in the second configuration, inserting the first single use wicking cartridge in the chamber. The method further includes, after inserting the at least one single use first wicking cartridge in the chamber, transforming the reusable fluid impermeable barrier from the second configuration into the first configuration.

In an embodiment, a system is disclosed. The system includes a fluid collection assembly. The fluid collection assembly includes a first end and a second end opposite the first end. The reusable fluid impermeable barrier includes at least one inner surface defining a chamber extending between the first end and the second end. The reusable fluid impermeable barrier also defines an outlet and at least one opening spaced from the outlet that both provide access to the chamber. The fluid collection assembly also includes at least one single use wicking cartridge configured to receive fluid. The reusable fluid impermeable barrier is configured to be transformed between a first configuration and a second configuration. The reusable fluid impermeable barrier does not allow the at least one single use wicking cartridge to be inserted and/or removed from the chamber when the reusable fluid impermeable barrier is in the first configuration and the reusable fluid impermeable barrier allows the at least one single use wicking cartridge to be inserted and/or removed from the chamber when the reusable fluid impermeable barrier is in the second configuration. The system also includes a fluid storage container positioned downstream from the fluid collection assembly, a vacuum source positioned downstream from the fluid storage container, at least one first tube extending between and fluidly coupling the fluid collection assembly to the fluid storage container, and at least one second tube extending between and fluid coupling the fluid storage container to the vacuum source.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIGS. 1A** and **1B** are isometric views of a fluid collection assembly that is in a first state and a second state, respectively, according to an embodiment.
**FIG. 1C** is a schematic cross-sectional view of the fluid collection assembly in the second state, as shown in **FIG. 1B****,** according to an embodiment.
**FIGS. 2A** to **2F** are schematic illustrations of a method of using a fluid collection assembly, according to an embodiment.
**FIGS. 3A-3D** are cross-sectional views of different single use wicking cartridges that may be used in any of the fluid collection assemblies disclosed herein, according to different embodiments.
**FIGS. 4A** and **4B** are isometric view of different fluid collection assembly in the first state having different markings, according to different embodiments.
**FIGS. 5A** and **5B** are isometric views of the inlet of tubes that have one or more slots formed therein, according to different embodiments.
**FIG. 6** is a schematic cross-sectional view of a fluid collection assembly that is configured to received urine from a male urethra, according to an embodiment.
**FIG. 7** is a schematic illustration of system that includes a fluid collection assembly, according to an embodiment.

### DETAILED DESCRIPTION

Fluid collection assemblies, methods of using the same, and systems including the same are disclosed herein. An example fluid collection assembly includes a reusable fluid impermeable barrier that is configured to be reusable and at least one single use wicking cartridge that may not be configured to be reusable. The reusable fluid impermeable barrier defines an chamber therein. The reusable fluid impermeable barrier and the single use wicking cartridge are configured to have the single use wicking cartridge disposed in a chamber defined by the reusable fluid impermeable barrier. The fluid collection assembly may also include at least one tube that is at least partially disposed in the chamber or otherwise in fluid communication with the chamber. The tube is configured to remove other bodily fluids *(e.g.,* urine) from the chamber. It is noted that the tube may also form part of a system that includes the fluid collection assembly.

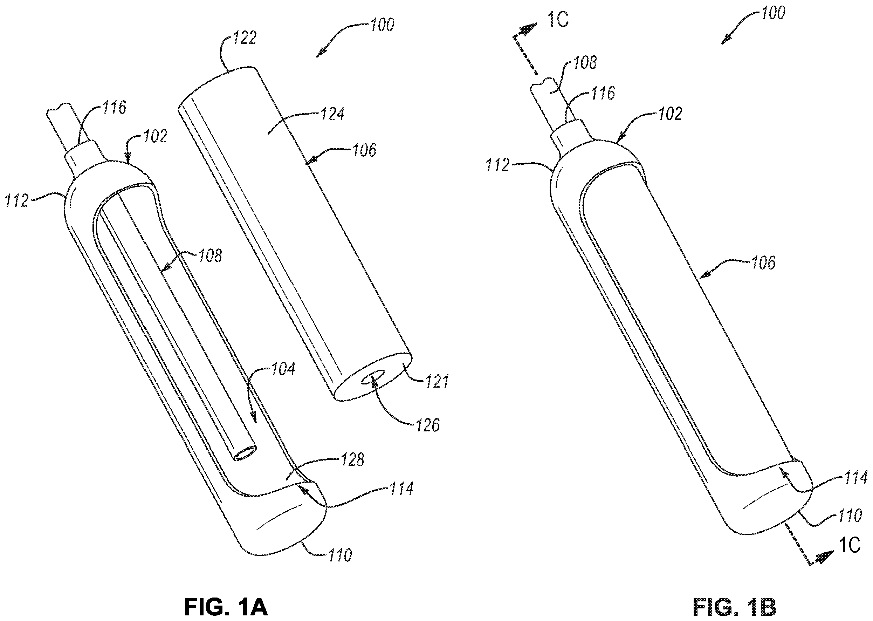

The fluid collection assembly is configured to be transformed between a first state and a second state. When the fluid collection assembly is in the first state, the single use wicking cartridge is not disposed in *(e.g.,* spaced from) the chamber of the reusable fluid impermeable barrier **(****FIG. 1A****).** In an embodiment, the fluid collection assembly may be in the first state initially *(e.g.,* before the fluid collection assembly is used) when the single use wicking cartridge is not initially disposed in the chamber, packaged separately from the reusable fluid impermeable barrier, etc. In an embodiment, the fluid collection assembly may be in the first state after a used single use wicking cartridge is removed from the reusable fluid impermeable barrier and before an unused single use wicking cartridge is positioned in the chamber.

The fluid collection assembly is in the second state when the single use wicking cartridge is disposed in the chamber **(****FIG. 1B****).** The fluid collection assembly may be in the second state at least when the fluid collection assembly is in use. The fluid collection assembly may in use when the fluid collection assembly is disposed adjacent to a urethral opening of a female user. In an embodiment, the fluid collection assembly is in the second state for a period of time before the fluid collection assembly is used. In such an embodiment, the single use wicking cartridge may be packaged in the chamber of the reusable fluid impermeable barrier or otherwise disposed in the chamber before the fluid collection assembly is used. In an embodiment, the fluid collection assembly is in the second state for a period of time after the fluid collection assembly is used. However, generally, the fluid collection assembly is in the second state for only a short period (*e.g.*, a few minutes or less) after the fluid collection assembly is used for sanitary reasons.

To transform the fluid collection assembly between the first and second states, the reusable fluid impermeable barrier may be transformable between a first configuration and a second configuration. When the reusable fluid impermeable barrier is in the first configuration, the reusable fluid impermeable barrier may prevent the single use wicking cartridge from being inserted into the chamber when the single use wicking cartridge is outside of the chamber and/or may prevent the single use wicking cartridge from being removed from the chamber when the single use wicking cartridge is inside of the chamber. When the reusable fluid impermeable barrier is in the second configuration, the single use wicking cartridge may be inserted and/or removed from the chamber.

In an embodiment, transforming the reusable fluid impermeable barrier between the first configuration and the second configuration includes changing the size of an opening of the reusable fluid impermeable barrier. For example, the opening of the reusable fluid impermeable barrier is closed or too small for the single use wicking cartridge to fit through when the reusable fluid impermeable barrier is in the first configuration while the opening of the reusable fluid impermeable barrier is sufficient large for the single use wicking cartridge to fit through when the reusable fluid impermeable barrier is in the second configuration. In an example, the reusable fluid impermeable barrier is transformed between the first configuration and the second configuration (*e.g.*, the opening of the reusable fluid impermeable barrier changes size) by bending, stretching, or otherwise modifying the shape of the reusable fluid impermeable barrier.

Configuring the fluid collection assembly to be transformed between the first and second states allows the reusable fluid impermeable barrier to be reused, such as using the reusable fluid impermeable barrier with a plurality of single use wicking cartridges. For example, the reusable fluid impermeable barrier is often formed from silicone, other thermosetting polymer, other type of polymer, or combinations thereof. In such an example, the reusable fluid impermeable barrier may pose several environmental concerns related to its poor biodegradability and the scarce natural resources used to form the reusable fluid impermeable barrier. However, reusing the reusable fluid impermeable barrier decreases the environmental effect of using the reusable fluid impermeable barrier, such as by reducing the amount of waste generated when using the fluid collection assembly. It also decreases the cost of using the fluid collection assembly.

In an embodiment, the reusable fluid impermeable barrier may be reused only with the same individual. For example, the reusable fluid impermeable barrier may be used with the same individual and may receive one or more *(e.g.,* a plurality of) single use wicking cartridges while being used with the individual. However, once the individual is done using the fluid collection assembly, the reusable fluid impermeable barrier is discarded. In an embodiment, the reusable fluid impermeable barrier may be used with multiple individuals. In such an embodiment, the reusable fluid impermeable barrier may be used with a first individual and may receive one or more *(e.g.,* a plurality of) single use wicking cartridges while being used by the first individual. Once the first individual is done using the fluid collection assembly, the reusable fluid impermeable barrier is washed, such as with a disinfectant (*e.g.*, at least one of antibacterial, antimicrobial, or antiseptic cleaner) and/or with an autoclave. Since the reusable fluid impermeable barrier is formed from silicone or other high temperature material, washing the reusable fluid impermeable barrier does not damage the reusable fluid impermeable barrier. The reusable fluid impermeable barrier may then be used with a second individual. It is noted that the reusable fluid impermeable barrier may be washed when the reusable fluid impermeable barrier is merely used with a single individual, such as when the reusable fluid impermeable barrier is used with the single individual for a long period of time and, for example, the growth of microbes in the reusable fluid impermeable becomes a concern.

**FIGS. 1A** and **1B** are isometric views of a fluid collection assembly 100 that is in the first state (*i.e.*, the single use wicking cartridge 106 is not disposed in the reusable fluid impermeable barrier 102) and a second state (*i.e.*, the single use wicking cartridge 106 is disposed in the reusable fluid impermeable barrier 102), respectively, according to an embodiment. The fluid collection assembly 100 includes a reusable fluid impermeable barrier 102 defining a chamber 104, a single use wicking cartridge 106, and optionally, at least one tube 108 in fluid communication with the chamber 104. The chamber 104 and the single use wicking cartridge 106 exhibit a size and shape that allows the single use wicking cartridge 106 to be disposed therein. In other words, the chamber 104 and the single use wicking cartridge 106 exhibits a size and shape *(e.g.,* a cross-sectional size and shape that is perpendicular to a longitudinal axis of the fluid collection assembly 100) that generally corresponds with each other.

The reusable fluid impermeable barrier 102 extends longitudinally between a first end 110 and an opposing second end 112. The reusable fluid impermeable barrier 102 defines at least one opening 114 positioned between the first and second ends 110, 112. The opening 114 is configured to be positioned adjacent to a female urethral opening and is configured to allow the female urethral opening to be in fluid communication with the chamber 104 when the opening 114 is positioned adjacent to the female urethral opening. For example, bodily fluids *(e.g.,* urine) that are excreted by the female urethral oppening enter the chamber 104 via the opening 114.

Generally, the opening 114 extends along a significant portion *(e.g.,* at least 20%, at least 50%, at least 75%, 20% to 60%, 50% to 75%, or 60% to 90%) of a length of the reusable fluid impermeable barrier 102 to increase the amount of fluid that enters the chamber 104. In an example, the opening 114 includes an elongated opening. In an example, the opening 114 includes a plurality of slits.

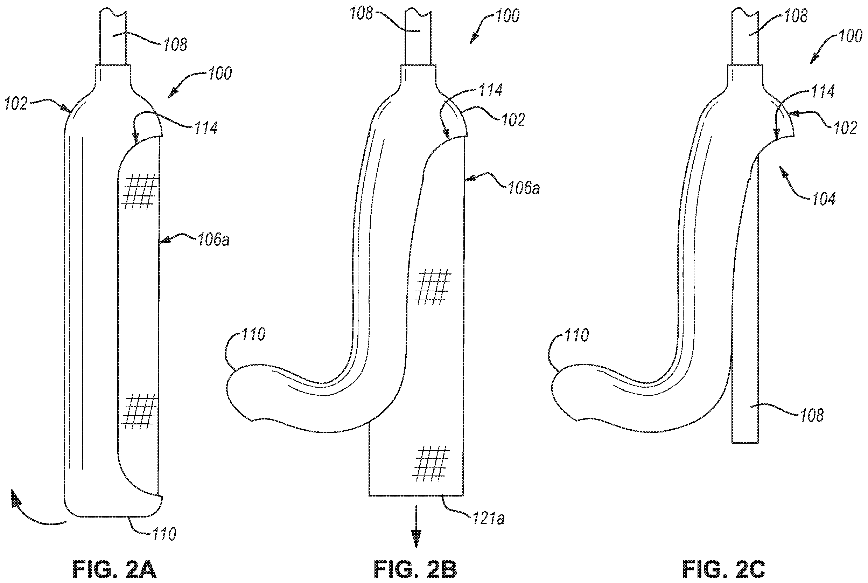

In an embodiment, as previously discussed, the opening 114 may be configured to change sizes thereof, thereby allowing the reusable fluid impermeable barrier 102 to be transformed between the first and second configurations. For example, as illustrated in **FIGS. 1A** and **1B****,** the reusable fluid impermeable barrier 102 is in the first configuration since the opening 114 does not exhibit a size that is sufficiently large to allow the single use wicking cartridge 106 to be inserted or removed from the chamber 104. However, as will be discussed in more detail with regards to **FIGS. 2A-2F****,** bending or otherwise modifying (*e.g.*, stretching) the reusable fluid impermeable barrier 102 may increase the size of the opening 114 such that the single use wicking cartridge 106 may be inserted or removed from the chamber 104. In an embodiment, the opening 114 may not be configured to change sizes. In such an embodiment, the reusable fluid impermeable barrier 102 may include another opening (not shown), such as an opening formed in the first end 110, that is configured to change sizes. For example, the opening formed in the first end 110 may be closed when the reusable fluid impermeable barrier 102 is in the first configuration to prevent fluid leaking from the chamber 104 through the opening formed in the first end 110.

The reusable fluid impermeable barrier 102 may be configured to bend or otherwise modify the shape thereof, thereby allowing the reusable fluid impermeable barrier 102 to be transformed between the first and second configurations. In an embodiment, at least a portion of the reusable fluid impermeable barrier 102 may be formed from a bendable and/or stretchable material, such as silicone or other suitable polymer thereby allowing the reusable fluid impermeable barrier 102 to bend and/or stretch. In an embodiment, the reusable fluid impermeable barrier 102 may include one or more hinges or other mechanical devices that allow the reusable fluid impermeable barrier 102 to bend or otherwise modify a shape thereof.

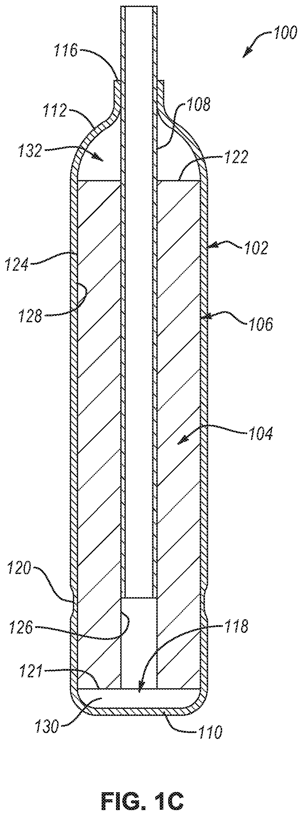

The reusable fluid impermeable barrier 102 may be configured to bend or otherwise modify the shape thereof in a specific location. In an embodiment, when the opening 114 is configured to change sizes, the specific location (*i.e.*, the portion of the reusable fluid impermeable barrier 102 configured to bend or otherwise modify a shape thereof) is located closer to the first end 110 than the second end 112. Selecting the specific location to be closer to the first end 110 than the second end 112 may make it easier to maneuver the first end 110 around a first tip 121 of the single use wicking cartridge 106 when the single use wicking cartridge 106 is positioned in the chamber 104 and to position the single use wicking cartridge 106 around the tube 108. In an example, the specific location may be more bendable *(e.g.,* flexible, stretchable, less rigid) than at least a portion of the reusable fluid impermeable barrier 102 that is between the specific location and the second end 112 which causes the reusable fluid impermeable barrier 102 to preferentially bend or otherwise modify a shape thereof at the specific location. For instance, the specific location may include a thinned region 120 (shown in FIG. 1C) that exhibits a thickness that is less than at least a portion of the reusable fluid impermeable barrier 102 that is between the specific location and the second end 112. Alternatively or in addition to the thinned region 120, the specific location may include a notch defined thereby, a recess defined thereby, seam formed therein, include a more bendable material than the rest of the reusable fluid impermeable barrier 102, or any other suitable mechanism that causes the specific location to preferentially bend or otherwise modify the shape thereof. It is noted that the first end 110 and/or portions of the reusable fluid impermeable barrier 102 between the specific location and the first end 110 may be more bendable than at least a portion of the reusable fluid impermeable barrier 102 between the specific location and the second end 112 *(e.g.,* equally or slightly less bendable than the specific location) to facilitate maneuvering the first end 110 around the first tip 121 of the single use wicking cartridge 106.

The reusable fluid impermeable barrier 102 also defines an outlet 116, for example, at the second end 112 thereof. The outlet 116 is configured to be coupled to and/or have the tube 108 disposed therein. As such, the outlet 116 allows the tube 108 to be in fluid communication with the chamber 104. In the illustrated embodiment, the tube 108 is also disposed in the chamber 104. In such an embodiment, the tube 108 may be positioned at or near a substantially empty fluid reservoir 118 (shown in FIG. 1C) which facilitates removal of any fluids *(e.g.,* urine) that are present in the fluid reservoir 118.

Referring to **FIG. 1A****,** the single use wicking cartridge 106 includes a first tip 121, a second tip 122 opposite the first tip 121, and at least one outer surface 124 extending between the first tip 121 and the second tip 122. The single use wicking cartridge 106 is configured to, when positioned in the chamber 104, receive fluids and wick the fluids away from the opening 114. For example, the single use wicking cartridge 106 may be configured to wick (especially in the presence of a suction force) the fluids towards an inlet of the tube 108 such that the fluids may be removed from the chamber 104. As such, the single use wicking cartridge 106 is generally formed from a porous material that allows the fluids to flow therethrough. The single use wicking cartridge 106 is formed from any suitable material that may receive *(e.g.,* absorb, adsorb, or wick) the fluids, such as cotton gauze. However, the single use wicking cartridge 106 is formed from a material that is configured to predominately wick (instead of predominately absorb or adsorb) the fluids. Examples of material that may predominately wick the fluids includes nylon *(e.g.,* spun nylon fibers) or polyolefins. In an embodiment, the single use wicking cartridge 106 is formed from non-woven material (*e.g.*, non-woven fibers) which may facilitate forming the single use wicking cartridge 106 via extrusion processes.

In an embodiment, the single use wicking cartridge 106 may include permeable material designed to wick or pass fluid therethrough. The permeable properties referred to herein may be wicking, capillary action, diffusion, or other similar properties or processes, and are referred to herein as "permeable" and/or "wicking." Such "wicking" may not include absorption of fluid into the wicking material. Put another way, substantially no absorption of fluid into the material may take place after the material is exposed to the fluid and removed from the fluid for a time. While no absorption is desired, the term "substantially no absorption" may allow for nominal amounts of absorption of fluid into the wicking material (*e.g.*, absorbency), such as less than about 10 wt% of the dry weight of the wicking material, less than about 7 wt%, less than about 5 wt%, less than about 3 wt%, less than about 2 wt%, less than about 1 wt%, or less than about 0.5 wt% of the dry weight of the wicking material.

Wicking material can include natural fibers. In such examples, the material may have a coating to prevent or limit absorption of fluid into the material, such as a water repellent coating.

During use, the single use wicking cartridge 106 may receive bodily fluids from the individual during a time period, as will be discussed in more detail below. After use, the single use wicking cartridge 106 is removed from the fluid impermeable barrier 102 and disposed of. The single use wicking cartridge 106 may not be reused because the single use wicking cartridge 106 needs to be cleaned to be reused. Cleaning the single use wicking cartridge 106 includes removing all of the bodily fluids from and sterilizing all of the surfaces of the single use wicking cartridge. However, the porosity of the single use wicking cartridge makes removing all of the bodily fluids and sterilizing all of the surfaces of the single use wicking cartridge difficult and costly. Further, cleaning of the single use wicking cartridge may damage the single use wicking cartridge 106 and increase the volume of the single use wicking cartridge 106 due to the extreme cleaning measures required to remove all of the bodily fluids from and sterilize all of the surfaces of the single use wicking cartridge. The increased volume of the single use wicking cartridge 106 may prevent insertion of the single use wicking cartridge 106 into the chamber 104.

When the tube 108 is disposed in the chamber 104, the single use wicking cartridge 106 defines at least one passageway 126 extending at least partially therethrough. For example, the second tip 122 defines an entrance of the passageway 126 (not shown, obscured). The passageway 126 exhibits a size and shape that allows the passageway 126 to receive the tube 108 *(e.g.,* the passageway 126 exhibits a size and shape that corresponds to or is slightly larger than the tube 108). The length of the passageway 126 depends on the length of the tube 108 that extends into the chamber 104. In particular, generally, the passageway 126 exhibits a length that is the same or greater than the length of a portion of the tube 108 that extends into the chamber 104. In an embodiment, the passageway 126 only extends partially through the single use wicking cartridge 106. In an embodiment, as illustrated, the passageway 126 extends completely through the single use wicking cartridge 106 such that the first tip 121 partially defines an opposing entrance of the passageway 126.

The outer surface 124 and the single use wicking cartridge 106, as a whole, may exhibit any suitable shape. For example, the outer surface 124 and the single use wicking cartridge 106 as a whole may exhibit a generally cylindrical shape (as shown), a generally box-like shape *(e.g.,* an elongated generally box-like shape), a generally triangular prism-like shape *(e.g.,* an elongated generally prism-like shape), a bent shape *(e.g.,* a bent generally cylindrical shape), or any other suitable shape. However, the shape and size of the outer surface 124 and the single use wicking cartridge 106 may depend on the size and shape of the chamber 104. For example, **FIG. 1C** is a schematic cross-sectional view of the fluid collection assembly 100 in the second state, as shown in **FIG. 1B****,** according to an embodiment. The reusable fluid impermeable barrier 102 includes at least one inner surface 128 that partially defines the chamber 104. At least a portion of the inner surface 128 of the reusable fluid impermeable barrier 102 and a corresponding portion of the outer surface 124 of the single use wicking cartridge 106 are selected to have a cross-sectional shape (measured perpendicular to the longitudinal axis of the reusable fluid impermeable barrier 102 and a parallel axis of the single use wicking cartridge 106) that correspond to each other. The cross-sectional shape of the inner surface 128 and the outer surface 124 correspond to each other when the cross-sectional shape of the outer surface 124 of the single use wicking cartridge 106 is the same size as, slightly larger *(e.g.,* at most 10%, at least most 5%, or at most 2% larger), or slightly smaller *(e.g.,* at most 10%, at most 5%, or at most 2% smaller) than the cross-sectional shape of the inner surface 128 of the reusable fluid impermeable barrier 102. Such cross-sectional shapes inhibit the formation of large air gaps between the inner surface 128 of the reusable fluid impermeable barrier 102 and the outer surface 124 of the single use wicking cartridge 106 which would cause pooling of fluids and/or inhibit the flow of the fluid towards the tube 108. Further, such cross-sectional shapes allow the single use wicking cartridge 106 to easily be inserted and/or removed from the chamber 104 since, at most, the single use wicking cartridge 106 is only lightly press-fitted into the reusable fluid impermeable barrier 102 when the fluid collection assembly 100 is in the second state.

In an embodiment, the single use wicking cartridge 106 may exhibit a length that is less than the length of the chamber 104. In such an embodiment, the fluid collection assembly 100 may define at least one of a first gap 130 at and/or near the first end 110 of the reusable fluid impermeable barrier 102 or a second gap 132 at and/or near the second end 112 of the reusable fluid impermeable barrier 102. The first and/or second gaps 130, 132 may facilitate the operation of the fluid collection assembly 100. For example, the first gap 130 may form a substantially unoccupied gap at and/or near the first end 110 of the reusable fluid impermeable barrier 102. Since, in operation, the first end 110 tends to be the low point of the fluid collection assembly 100, fluid is likely to collect in the first gap 130 before being removed from the chamber 104 via the tube 108. As such, the first gap 130 forms part of the fluid reservoir 118. Further, the first gap 130 makes it easier to maneuver the first end 110 of the reusable fluid impermeable barrier 102 around the first tip 121 of the single use wicking cartridge 106 when transforming the reusable fluid impermeable barrier 102 between the first and second configurations thereof.

Further, as shown in **FIG. 1C****,** the chamber 104 at and/or near the second end 112 of the reusable fluid impermeable barrier 102 generally tapers *(e.g.,* the diameter of the inner surface 128 decreases), for example, towards the outlet 116. The single use wicking cartridge 106 may not exhibit a shape that similarly tapers since, for example, the single use wicking cartridge 106 may be formed via extrusion. As such, causing the single use wicking cartridge 106 to exhibit a length that is less than the chamber 104 (thereby forming the second gap 132) indicates that the single use wicking cartridge 106 does not have to be inserted or completely inserted into the tapered portion of the chamber 104. Thus, the second gap 132 prevents the second tip 122 of the single use wicking cartridge 106 from being compressed by the tapered portion of the chamber 104 when the single use wicking cartridge 106 is not similarly tapered . For example, compressing the second tip of the single use wicking cartridge 106 may inhibit easy insertion and/or removal of the single use wicking cartridge 106 into the chamber 104.

**FIGS. 2A** to **2F** are schematic illustrations of a method of using a fluid collection assembly, according to an embodiment. The fluid collection assembly illustrated in **FIGS. 2A-2F** is the fluid collection assembly 100 illustrated in **FIGS. 1A-1C**. However, it is noted that the method illustrated in **FIGS. 2A-2F** may be used with any of the fluid collection assemblies disclosed herein.

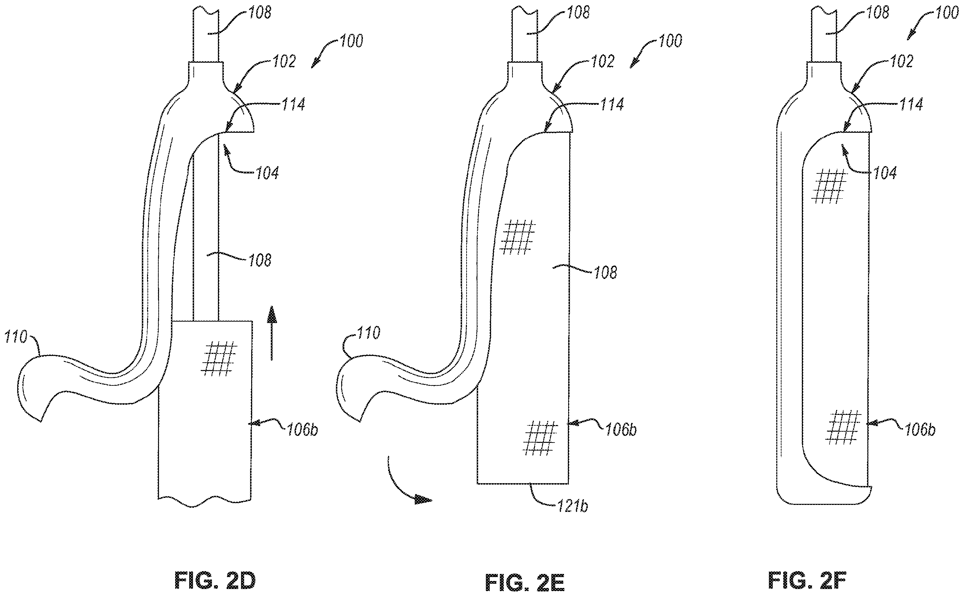

Referring to **FIG. 2A**, a fluid collection assembly 100 is provided. The fluid collection assembly 100 includes a reusable fluid impermeable barrier 102 and a first single use wicking cartridge 106a. In an embodiment, as shown, the fluid collection assembly 100 is provided in the second state, namely that the first single use wicking cartridge 106a is disposed in the chamber 104 (shown in **FIGS. 2C** and **2D****)** defined by the reusable fluid impermeable barrier 102. However, in an embodiment, the fluid collection assembly 100 may be initially provided in the first state, namely that the first single use wicking cartridge 106a is initially spaced from the chamber 104. In such an embodiment, the method includes inserting the first single use wicking cartridge 106a into the reusable fluid impermeable barrier 102, as generally shown in **FIGS. 2D-2F****.**

The fluid collection assembly 100 may be initially provided while the first single use wicking cartridge 106a is in an unused condition *(e.g.,* the first single use wicking cartridge 106a has not receive any fluids). The fluid collection assembly 100 may then be positioned such that the opening 114 is adjacent to a urethral opening *(e.g.,* the opening 114 of the illustrated fluid collection assembly 100 is configured to be adjacent to a female urethral opening), thereby allowing the first single use wicking cartridge 106a to receive bodily fluids from the urethral opening. Receiving bodily fluids from the urethral opening causes the first single use wicking cartridge 106a to be in a used condition. In an embodiment, the first single use wicking cartridge 106a may be removed from the chamber 104, for example, after the first single use wicking cartridge 106a is in the used condition or at some time after the first single use wicking cartridge 106a initially enters the used condition. In an embodiment, the first single use wicking cartridge 106a may be removed from the chamber 104 at some predetermined time after positioning the opening 114 adjacent to the urethral opening for sanitary reasons and/or to reduce clogging caused residual material deposited in the first single use wicking cartridge 106a by the fluids. The predetermined time may be at least about 1 hour, at least about 2 hours, at least about 4 hours, at least about 6 hours, at least about 8 hours, at least about 10 hours, at least about 12 hours, at least about 14 hours, at least about 16 hours, at least about 18 hours, at least about 20 hours, at least about 22 hours, at least about 24 hours, or in ranges of about 1 hour to about 4 hours, about 2 to about 6 hours, about 4 to about 8 hours, about 6 hours to about 10 hours, about 8 hours to about 12 hours, about 10 hours to about 14 hours, about 12 hours to about 16 hours, about 14 hours to about 18 hours, about 16 hours to about 20 hours, about 18 hours to about 22 hours, or about 20 hours to about 24 hours.

**FIGS. 2B** and **2C** illustrate how the first single use wicking cartridge 106a is removed from the chamber 104. Referring to **FIG. 2B****,** the reusable fluid impermeable barrier 102 is transformed from the first configuration into the second configuration. In the illustrated embodiment, the reusable fluid impermeable barrier 102 is transformed from the first configuration into the second configuration by bending a portion of the reusable fluid impermeable barrier 102 at a location that is at or near the first end 110 and maneuvering the first end 110 around the first tip 121a of the first single use wicking cartridge 106a. Once the reusable fluid impermeable barrier 102 is in the second configuration, the first single use wicking cartridge 106a may be slid along the tube 108 (if the tube 108 is disposed in the chamber 104) and easily removed from the chamber 104, as shown in **FIG. 2C****.** The first single use wicking cartridge 106a may be disposed of in any suitable manner.

Referring to **FIG. 2D****,** if the reusable fluid impermeable barrier 102 is to used again, a second single use wicking cartridge 106b may be positioned in the chamber 104. For example, the second single use wicking cartridge 106b may be provided that is in the unused condition. The second single use wicking cartridge 106b may be the same or substantially similar to any of the single use wicking cartridges disclosed herein *(e.g.,* may be the same or substantially similar to the first single use wicking cartridge 106a). While the reusable fluid impermeable barrier 102 is in the second configuration, the second single use wicking cartridge 106b may be inserted into the chamber 104. For instance, in the illustrated embodiment, the second single use wicking cartridge 106b may inserted in the chamber 104 by positioning the tube 108 in a passageway (not shown) defined by the second single use wicking cartridge 106b and then sliding the second single use wicking cartridge 106b along the tube 108 until the second single use wicking cartridge 106b is correctly positioned into the chamber 104.

Once the second single use wicking cartridge 106b is correctly positioned into the chamber 104, the reusable fluid impermeable barrier 102 is transformed from the second configuration into the first configuration. For example, in the illustrated embodiment, the reusable fluid impermeable barrier 102 is transformed from the first configuration into the second configuration by bending the reusable fluid impermeable barrier 102 and maneuvering the first end 110 of the reusable fluid impermeable barrier 102 around the first tip 121b of the second single use wicking cartridge 106b, as shown in **FIG. 2E****.** Then, the fully assembled fluid collection assembly 100 is ready to be used (*e.g.*, the opening 114 thereof is ready to be positioned adjacent to a urethral opening), as shown in **FIG. 2F****.**

It is noted that the method may include additional steps not illustrated in **FIGS. 2A-2F****.** For example, the method may include washing the reusable fluid impermeable barrier 102 between the acts illustrated in **FIGS. 2C** and **2D****.** Washing the reusable fluid impermeable barrier 102 may include merely rinsing the reusable fluid impermeable barrier to remove at least some of the fluids that may remain on the reusable fluid impermeable barrier 102 after removing the first single use wicking cartridge 106a and/or may include at least partially sterilizing the reusable fluid impermeable barrier 102. Sterilizing the reusable fluid impermeable barrier 102 may include washing the reusable fluid impermeable barrier 102 with a disinfectant (*e.g.*, anti-bacterial soap) or autoclaving the reusable fluid impermeable barrier 102.

In embodiment, the method illustrated in **FIGS. 2A-2F** may be repeated as many times as necessary. For example, the method illustrated in **FIGS. 2A-2F** may be repeated to remove the second single use wicking cartridge 106b and to add an additional single use wicking cartridge. Further, the methods illustrated in **FIGS. 2A-2F** may be repeated again to remove the additional single use wicking cartridge, and so forth.

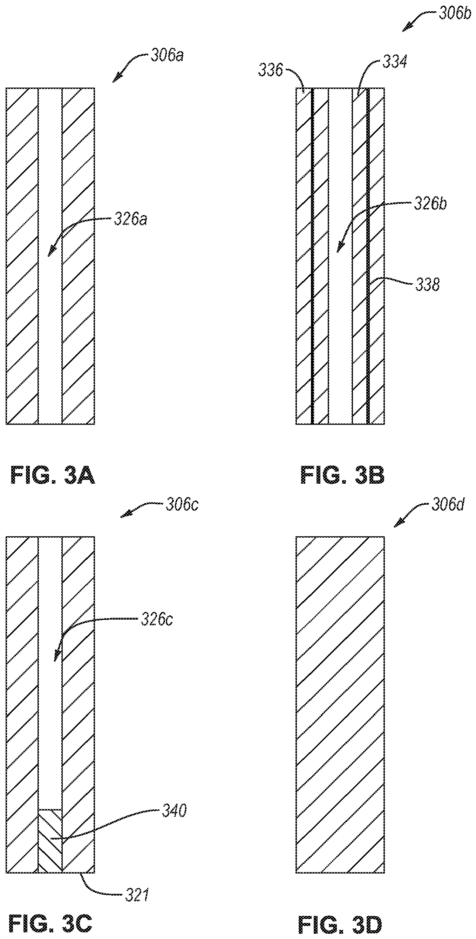

**FIGS. 3A-3D** are cross-sectional views of different single use wicking cartridges that may be used in any of the fluid collection assemblies disclosed herein, according to different embodiments. Except as otherwise disclosed herein, the single use wicking cartridges illustrated in **FIGS. 3A-3D** are the same or substantially similar to any of the single use wicking cartridges disclosed herein (including each other). For example, the single use wicking cartridges may include a first tip, an opposing second tip, an outer surface extending between the first and second tips, and, optionally, a passageway.

Referring to **FIG. 3A****,** the single use wicking cartridge 306a is formed from a single component *(e.g.,* is unitary) and defines a passageway 326a. The single use wicking cartridge 306a also exhibits a thickness that is substantially uniform along an entire length thereof. The single use wicking cartridge 306a may exhibit the substantially uniform thickness because the single use wicking cartridge 306a is formed using extrusion. However, it is noted that the single use wicking cartridge 306a may be formed using any suitable methods other than extrusion, such as rolling a sheet of porous material and bonding *(e.g.,* with an adhesive) the opposing ends of the sheet together. It is also noted that any of the single use wicking cartridges disclosed herein can exhibits a thickness that is non-uniform.

Referring to **FIG. 3B****,** the single use wicking cartridge 306b is formed from two or more components. For example, the single use wicking cartridge 306b may include a fluid permeable support 334 and a fluid permeable membrane 336. The fluid permeable support 334 may define the passageway 326b and may support the fluid permeable membrane 336 above the passageway 326b. The fluid permeable support 334 may also support the fluid permeable membrane 336 when the fluid permeable membrane 336 extends across the opening of a reusable fluid impermeable barrier. In an embodiment, the fluid permeable membrane 336 is selected to be more comfortable against a female urethra than the fluid permeable support 334. In an embodiment, the fluid permeable support 334 is selected better wick fluid *(e.g.,* is less absorbent or adsorbent) than the fluid permeable membrane 336. In either embodiment, the fluid permeable membrane 336 may be selected to be gauze or cloth and the fluid permeable support 334 may be selected spun nylon fibers and/or polyolefin. It is noted that the fluid permeable membrane 336 may include materials other than gauze or cloth and the fluid permeable support 334 may include materials other than spun nylon fibers or polyolefin and that these materials are only provided as examples.

The single use wicking cartridge 306b may also include an adhesive layer 338 between at least a portion of the fluid permeable support 334 and a corresponding portion of the fluid permeable membrane 336. The adhesive layer 338 maintains the positions of the fluid permeable support 334 and the fluid permeable membrane 336 relative to each other when the single use wicking cartridge 306b is inserted and/or removed from a chamber. For example, without the adhesive layer 338, the fluid permeable support 334 and the fluid permeable membrane 336 may be susceptible to move relative to each other which may make insertion and/or removal of the single use wicking cartridge 306b more difficult. Further, allowing the fluid permeable support 334 and the fluid permeable membrane 336 to move relative to each other may form non-desirous air gaps which may allow the fluid to pool and stagnate.

The single use wicking cartridge 306b may be formed using any suitable method. In an example, the single use wicking cartridge 306b may be formed using a dual extrusion process. In an example, the single use wicking cartridge 306b may be formed by rolling or extruding the fluid permeable support 334, and then positioning the fluid permeable membrane 336 around the fluid permeable support 334. The method of forming the single use wicking cartridge 306 may also include applying an adhesive to at least a portion of the exterior surface of the fluid permeable support 334 and positioning the fluid permeable membrane 336 around the fluid permeable support 334 also includes positioning the fluid permeable membrane 336 around the adhesive layer 338.

Referring to **FIG. 3C****,** the single use wicking cartridge 306c defines a passageway 326c that only extends partially through the single use wicking cartridge 306c. The passageway 326c may only extend partially through the single use wicking cartridge 306c when, for example, a tube *(e.g.,* tube 108 of **FIG. 1A****)** does not extend to or near the fluid reservoir. In an embodiment, the tube may only extend partially through the single use wicking cartridge 306c because, for example, the tube may impede transforming the reusable fluid impermeable barrier between the first and second configurations when the tube extends completely through the single use wicking cartridge 306c. Only partially extending the tube through the single use wicking cartridge 306c may cause the tube to be spaced from a fluid reservoir (*e.g.*, fluid reservoir 118 shown in **FIG. 1C****)** of the fluid collection assembly which may inhibit the tube receiving the fluids. To remedy this problem, the single use wicking cartridge 306c may include a porous portion positioned between the passageway 326c and the first tip 321 of the single use wicking cartridge 306b ("porous portion"). The porous portion may be configured to wick fluid from the first tip 321 *(e.g.,* from fluid reservoir) to the passageway 326c thus allowing the tube to receive fluids that are present in the fluid reservoir.

In an embodiment, the porous portion of the single use wicking cartridge 306c is integrally formed with at least one other component of the single use wicking cartridge 306c. However, the porous portion that is integrally formed with at least one other component of the single use wicking cartridge 306c may be difficult to form depending on the method used to form the single use wicking cartridge 306c. For example, the porous portion that is integrally formed with at least one other component of the single use wicking cartridge 306c may be difficult to form when the single use wicking cartridge 306c is formed by extrusion. As such, in an embodiment, the single use wicking cartridge 306c may include a plug 340 *(e.g.,* the porous portion is the plug 340) that is distinct from the rest of the single use wicking cartridge 306c. The plug 340 may be positioned such that the plug 340 extends between the passageway 326c and the first tip 321 and the plug 340 wicks material from the first tip 321 to the passageway 326c. The plug 340 may exhibit a size that allows the plug 340 to be inserted into the passageway 326c. The plug 340 may be formed from the same or different material than the rest of the single use wicking cartridge 306c. In an example, the plug 340 may be adhesively attached to the rest of the single use wicking cartridge 306c.

Referring to **FIG. 3D****,** the single use wicking cartridge 306d does not define a passageway. The single use wicking cartridge 306d does not define a passageway, for example, when the tube is not positioned in the chamber or only extends for a short distance into the chamber *(e.g.,* only extends into the second gap 132 shown in **FIG. 1C****).**

In some embodiments, correct positioning of the single use wicking cartridge in the reusable fluid impermeable barrier may be important. In an example, inserting the single use wicking cartridge too far into the reusable fluid impermeable barrier may at least one of form an inefficiently large first gap that causes excessive pooling and stagnation of the fluids or may compress the portions of the single use wicking cartridge near the second end of the reusable fluid impermeable barrier which may make removal of the single use wicking cartridge difficult. In an example, failing the completely insert the singe use wicking cartridge into the reusable fluid impermeable barrier may at least one of prevent the formation of the fluid reservoir, make transforming the reusable fluid impermeable barrier between the first and second configurations difficult, or cause the formation of an inefficiently large second gap that causes excessive pooling and stagnation of the fluids. As such, the single use wicking cartridges disclosed herein may include one or more features that facilitate correct placement of the single use wicking cartridges.

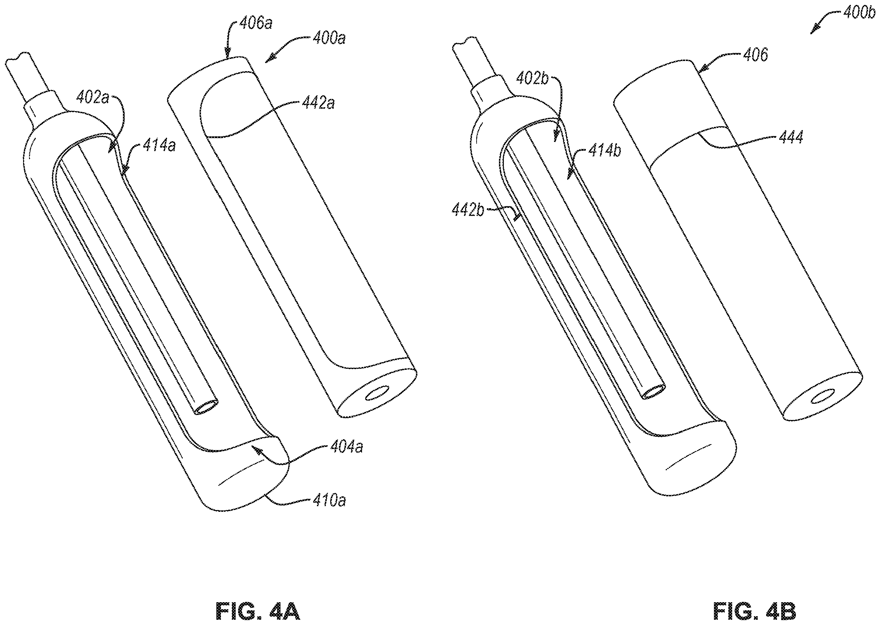

In an embodiment, the features that facilitate the correct placement of the single use wicking cartridges in the reusable fluid impermeable barrier includes one or more markings formed on at least one of the reusable fluid impermeable barrier or the single use wicking cartridge. The markings on one of the reusable fluid impermeable barrier or the single use wicking cartridge may correspond to features *(e.g.,* other markings) on the other of the reusable fluid impermeable barrier or the single use wicking cartridge. **FIGS. 4A** and **4B** are isometric view of different fluid collection assembly in the first state having different markings, according to different embodiments. Except as otherwise disclosed herein, the fluid collection assembly illustrated in **FIGS. 4A** and **4B** are the same or substantially similar to any of the fluid collection assemblies disclosed herein.

Referring to **FIG. 4A****,** the fluid collection assembly 400a includes a reusable fluid impermeable barrier 402a defining an opening 414a. The fluid collection assembly 400a also includes a single use wicking cartridge 406a. The single use wicking cartridge 406a includes at least one marking 442a formed thereon. The marking 442a may include a line and/or a shaded region. The marking 442a may exhibit a shape of at least a portion of the opening 414a. In the illustrated embodiment, the marking 442a exhibits the shape of an entirety of the opening 414a. However, it is noted that the marking 442a may only correspond to a portion of the opening 414a. For example, the marking 442a may only correspond to a portion of the opening 414a near the second end 412 since the portion of the reusable fluid impermeable barrier 402a near the first end 410a may be bent or otherwise deformed when the reusable fluid impermeable barrier 402a is in the second configuration.

Referring to **FIG. 4B****,** the fluid collection assembly 400b includes a reusable fluid impermeable barrier 402b and a single use wicking cartridge 406b. The reusable fluid impermeable barrier 402b includes a first marking 442b and the single use wicking cartridge 406b includes a second marking 444. The first and second markings 442b, 444 are located on respective portions of the reusable fluid impermeable barrier 402b and the single use wicking cartridge 406b such that the single use wicking cartridge 406b is correctly positioned when the first and second markings 442b, 444 align.

To facilitate correct positioning the single use wicking cartridge 406b, the first marking 442b may be located adjacent to opening 414b of the reusable fluid impermeable barrier 402b. The first marking 442b may also be located nearer the second end 412b than the first end 410b of the reusable fluid impermeable barrier 402b such that bending or otherwise modifying the shape of the reusable fluid impermeable barrier 402b is unlikely to change the location of the first marking 442b.

In an embodiment, as illustrated, the second marking 444 extends around a circumference of the singe use wicking cartridge 406b. This allows the single use wicking cartridge 406b to be correctly positioned regardless of the rotation of the single use wicking cartridge 406b. However, it is noted that the second marking 444 may not extend around the circumference of the single use wicking cartridge 406b, for example, when the single use wicking cartridge 406b cannot be freely rotated relative to the reusable fluid impermeable barrier 402b.

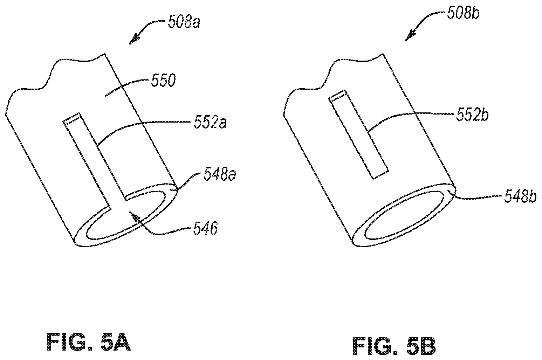

Improper placement of the single use wicking cartridge in the chamber may cause the single use wicking cartridge to block or otherwise obscure an inlet of a tube. In an example, inserting the single use wicking cartridge too far into the chamber may cause a porous portion *(e.g.,* plug 340 shown in **FIG. 3C****)** of the single use wicking cartridge to block or otherwise obscure the inlet of the tube. In an example, not inserting the single use wicking cartridge sufficiently far into the chamber may cause the single use wicking cartridge to bunch up at or near the first tip thereof, which may lead to the collapse of any passageway thereof thereby blocking or otherwise obscuring the inlet of the tube. Blocking or otherwise obscuring the inlet of the tube may decrease the functionality of the fluid collection assembly since blocking or obscuring the inlet of the tube reduces or inhibits the flow of fluid out of the chamber. To mitigate this problem, the tube may include one or more slots formed therein that are less likely to be blocked or obscured by incorrect placement of the single use wicking cartridge. **FIGS. 5A** and **5B** are isometric views of the inlet of tubes that have one or more slots formed therein, according to different embodiments. The tubes illustrated in **FIGS. 5A-5B** may be used in any of the fluid collection assembly disclosed herein.

Referring to **FIG. 5A****,** the tube 508a defines an inlet 546 at a terminal end 548a thereof and at least one wall 550 extending from the terminal end 548a. The tube 508a also defines at least one slot 552a extending from the terminal end 548a for any suitable distance. It is noted that the at least one slot 552a does not have to extend from the terminal end 548a. For example, as illustrated in **FIG. 5B****,** the tube 508b may include at least one slot 552b that is spaced above the terminal end 548b. Spacing the slot 552b above the terminal end 548b may decrease the likelihood that the slot 552b becomes obscured or blocked.

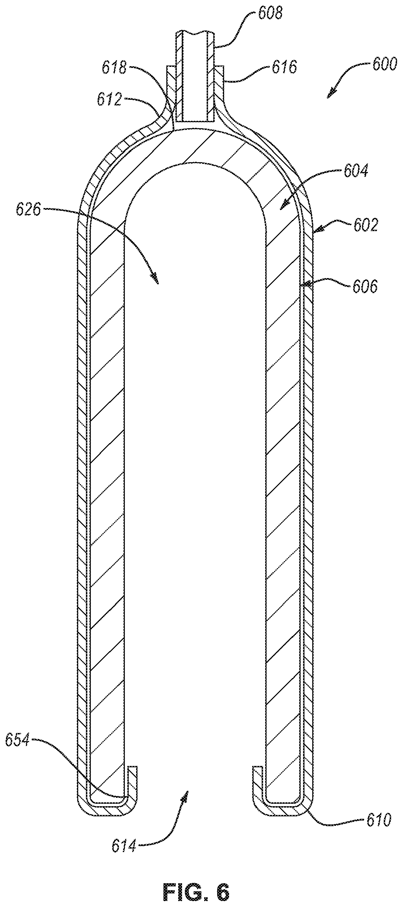

The features and principles regarding the fluid collection assemblies disclosed above may also be applied to a fluid collection assembly that is configured to receive urine from a male urethral opening *(e.g.,* penis). For example, **FIG. 6** is a schematic cross-sectional view of a fluid collection assembly 600 that is configured to received urine from a male urethra, according to an embodiment. Except as otherwise disclosed herein, the fluid collection assembly 600 is the same or substantially similar to any of the fluid collection assemblies disclosed herein. For example, the fluid collection assembly 600 includes a reusable fluid impermeable barrier 602 that defines a chamber 604 and a single use wicking cartridge 606. The fluid collection assembly 600 may also include at least one tube 608 that is in fluid communication with the chamber 604.

The reusable fluid impermeable barrier 602 includes a first end 610 and an opposing second end 612. The first end 610 of the reusable fluid impermeable barrier 602 defines an opening 614 and the second end 612 of the reusable fluid impermeable barrier 602 may define the outlet 616 that is in fluid communication with the tube 608 *(e.g.,* the tube 608 is positioned through the outlet 616). The opening 614 and the outlet 616 provide access to the chamber 604. As illustrated, the opening 614 is configured to receive the male urethral opening such that the male urethral opening is disposed in the chamber 604. However, it is noted that, in some embodiments, the opening 614 may be configured to be positioned adjacent to the male urethral opening *(e.g.,* buried penis) instead of receiving the male urethral opening.

The reusable fluid impermeable barrier 602 is configured to be transformed between a first configuration and a second configuration. In the illustrated embodiment, the reusable fluid impermeable barrier 602 may include at least one flap 654 at the first end 610 thereof. The flap 654 may be configured to adjust the size of the opening 614. For example, the flap 654 may be configured to curve inwardly when the reusable fluid impermeable barrier 602 is in the first configuration thereby reducing the size of the opening 614 and preventing the single use wicking cartridge 606 from being inserted and/or removed from the chamber 604, as illustrated. However, the flap 654 may be flexible and/or stretchable such that the flap 654 may be configured to curve outwardly with some manipulation when the reusable fluid impermeable barrier 602 is in the second configuration thereby increasing the size of the opening 614 such that the single use wicking cartridge 606 may be inserted and/or removed from the chamber 604. It is noted that other mechanisms may be used instead of the flap 654. For example, the reusable fluid impermeable barrier 602 may include a cap or pin that can be used to be transformed the reusable fluid impermeable barrier 602 between the first and second configurations.

The single use wicking cartridge 606 may be substantially similar to any of the single use wicking cartridges disclosed herein. In an example, the single use wicking cartridge 606 is configured to wick fluids towards a fluid reservoir 618 that is located at or near the second end 612 of the reusable fluid impermeable barrier 602. In an example, as illustrated, the single use wicking cartridge 606 may include a single material, similar to the single use wicking cartridge 306a of FIG. 3A. However, the single wick wicking cartridge 606 may include two or more materials, similar to the single use wicking cartridge 306b of **FIG. 3B****.** In an example, the single use wicking cartridge 606 defines a passageway 626. However, the passageway 626 is sized and shaped to receive a male urethral opening. Further, the passageway 626 may only extend partially through the single use wicking cartridge 606 such that a porous portion of the single use wicking cartridge 606 is between the male urethral opening and the outlet 616. Only partially extending the passageway 626 through the single use wicking cartridge 606 prevents the male urethral opening from being suctioned to the outlet 616.

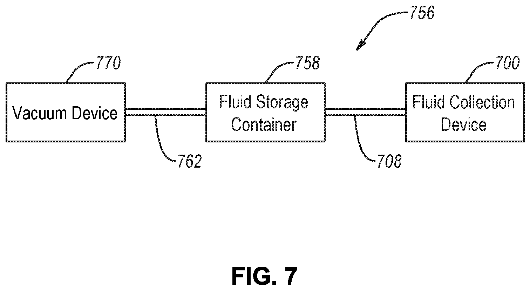

**FIG. 7** is a schematic illustration of system 756 that includes a fluid collection assembly 700, according to an embodiment. The fluid collection assembly 700 may include any of the fluid collection assemblies disclosed herein. The fluid collection assembly 700 may be in fluid communication with a fluid storage container 758 via at least one first tube 708 *(e.g.,* tube 108, 608 of **FIGS. 1A** and **6****).** The fluid storage container 758 is positioned downstream from the fluid collection assembly 700. The fluid storage container 758 may be in fluid communication with a vacuum source 760 via at least one second tube 762. The vacuum source 760 is positioned downstream from the fluid storage container 758. During operation, the vacuum source 760 provides a suction force to the fluid collection assembly 700. The suction force draws fluid into the chamber and towards the first tube 708. The fluid that enters the first tube 708 is pulled by the suction force towards the fluid storage container 758 such that the fluid storage container 758 receives the fluid. The fluid storage container 758 may be configured to inhibit the fluid from flowing from the fluid storage container 758 to the vacuum source 760.

While various aspects and embodiments have been disclosed herein, other aspects and embodiments are contemplated. The various aspects and embodiments disclosed herein are for purposes of illustration and are not intended to be limiting.

Terms of degree *(e.g.,* "about," "substantially," "generally," etc.) indicate structurally or functionally insignificant variations. In an example, when the term of degree is included with a term indicating quantity, the term of degree is interpreted to mean ± 10%, ±5%, +2% or 0% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc. The present invention is set out in the appended claims. The embodiments, examples or aspects according to the present description which do not fall within the scope of said claims are provided for illustrative purposes only and do not form part of the present invention.

## Claims

1. A fluid collection assembly (100), comprising:
a washable and reusable fluid impermeable barrier (102) formed from a bendable stretchable material including a first end and a second end opposite the first end, the reusable fluid impermeable barrier including at least one inner surface defining a chamber (104) extending between the first end (110) and the second end (112), the reusable fluid impermeable barrier also defining an outlet (116) and at least one opening (114) spaced from the outlet that both provide access to the chamber; and
at least one single use wicking cartridge (106) configured to receive fluid;
wherein the reusable fluid impermeable barrier is configured to be transformed by bending and stretching between a first configuration (Figs. 2A and F) and a second configuration (Figs. 2B - E), wherein the reusable fluid impermeable barrier does not allow the at least one single use wicking cartridge to be inserted and/or removed from the chamber when the reusable fluid impermeable barrier is in the first configuration and the reusable fluid impermeable barrier allows the at least one single use wicking cartridge to be inserted into and removed from the chamber through the opening when the reusable fluid impermeable barrier is in the second configuration.

2. The fluid collection assembly of claim 1, wherein the reusable fluid impermeable barrier includes a specific location that is closer to the first end than the second end, the specific location is bendable, stretchable and shape modifiable, and wherein the reusable fluid impermeable barrier transforms from the first configuration into the second configuration responsive to bending and stretching the specific location.

3. The fluid collection assembly of claims 1 or 2, wherein the reusable fluid impermeable barrier and the at least one single use wicking cartridge define at least one gap between the at least one single use wicking cartridge and the first end, and/or
the reusable fluid impermeable barrier and the at least one single use wicking cartridge define at least one gap between the at least one single use wicking cartridge and the second end.

4. The fluid collection assembly of any one of the preceding claims, further comprising at least one tube (108) at least partially disposed in the chamber; and wherein the at least one single use wicking cartridge defines a passageway extending at least partially therethrough that is configured to receive the at least one tube.

5. The fluid collection assembly of claim 4, wherein the at least one tube includes one or more slots formed therein.

6. The fluid collection assembly of either of claims 4 and 5, wherein the passageway extends completely through the at least one single use wicking cartridge.

7. The fluid collection assembly of any one of claims 4-6, wherein the at least one single use wicking cartridge further includes a plug disposed in the passageway.

8. The fluid collection assembly of any one of the preceding claims, wherein the at least one single use wicking cartridge includes a fluid permeable support, a fluid permeable membrane, and an adhesive layer disposed between at least a portion of the fluid permeable support and a corresponding portion of the fluid permeable membrane.

9. The fluid collection assembly of any one of the preceding claims, wherein the at least one single use wicking cartridge includes one or more markings thereon, the one or more markings corresponding to one or more features of the reusable fluid impermeable barrier.

10. The fluid collection assembly of claim 9, wherein the one or more features of the reusable fluid impermeable barrier include one or more additional markings thereon.

11. The fluid collection assembly of any one of the preceding claims, wherein the at least one single use wicking cartridge including at least one outer surface exhibiting a size and shape that corresponds to size and shape of at least a portion of at least one inner surface of the reusable fluid impermeable barrier.

12. The fluid collection assembly of any one of the preceding claims, wherein the at least one single use wicking cartridge is at least partially formed from a non-woven material.

13. A method of using the fluid collection assembly according to any of claims 1-12, the method comprising:
providing the fluid collection assembly including a reusable fluid impermeable barrier and a first single use wicking cartridge, wherein:
the reusable fluid impermeable barrier includes a first end and a second end opposite the first end, the reusable fluid impermeable barrier including at least one inner surface defining a chamber extending between the first end and the second end, the reusable fluid impermeable barrier also defining an outlet and at least one opening spaced from the outlet that both provide access to the chamber, wherein the reusable fluid impermeable barrier is configured to be transformed between a first configuration and a second configuration;
the first single use wicking cartridge is configured to receive fluid;
with the reusable fluid impermeable barrier is in the second configuration, inserting the first single use wicking cartridge in the chamber; and
after inserting the at least one single use first wicking cartridge in the chamber, transforming the reusable fluid impermeable barrier from the second configuration into the first configuration.

14. The method of claim 13, wherein inserting the first single use wicking cartridge in the chamber includes positioning at least one tube disposed in the chamber in a passageway defined by the first single use wicking cartridge.

15. The method of either of claims 13 and 14, further comprising, after transforming the reusable fluid impermeable barrier from the second configuration into the first configuration:
transforming the reusable fluid impermeable barrier from the first configuration into the second configuration;
removing the first single use wicking cartridge from the chamber; and further comprising, after removing the first single use wicking cartridge from the chamber:
inserting a second single use wicking cartridge into the chamber; and
transforming the reusable fluid impermeable barrier from the second configuration into the first configuration.

## Patentansprüche

1. Fluidsammelanordnung (100), die Folgendes umfasst:
eine waschbare und wiederverwendbare fluidundurchlässige Barriere (102), die aus einem biegsamen, dehnbaren Material gebildet ist, die ein erstes Ende und ein zweites Ende, das dem ersten Ende entgegengesetzt ist, beinhaltet, wobei die wiederverwendbare fluidundurchlässige Barriere mindestens eine Innenoberfläche beinhaltet, die eine Kammer (104) definiert, die sich zwischen dem ersten Ende (110) und dem zweiten Ende (112) erstreckt, wobei die wiederverwendbare fluidundurchlässige Barriere auch einen Auslass (116) und mindestens eine Öffnung (114), die von dem Auslass beabstandet ist, die beide Zugang zu der Kammer bereitstellen, definiert; und
mindestens eine Einweg-Dochtpatrone (106), die dazu konfiguriert ist, Fluid aufzunehmen;
wobei die wiederverwendbare fluidundurchlässige Barriere dazu konfiguriert ist, durch Biegen und Dehnen zwischen einer ersten Konfiguration (Fig. 2A und F) und einer zweiten Konfiguration (Fig. 2B - E) umgewandelt zu werden, wobei die wiederverwendbare fluidundurchlässige Barriere nicht erlaubt, dass die mindestens eine Einweg-Dochtpatrone eingesetzt und/oder aus der Kammer entfernt wird, wenn die wiederverwendbare fluidundurchlässige Barriere in der ersten Konfiguration ist, und die wiederverwendbare fluidundurchlässige periphere erlaubt, dass die mindestens eine Einweg-Dochtpatrone in die Kammer durch die Öffnung eingesetzt und daraus entfernt wird, wenn die wiederverwendbare fluidundurchlässige Barriere in der zweiten Konfiguration ist.

2. Fluidsammelanordnung nach Anspruch 1, wobei die wiederverwendbare fluidundurchlässige Barriere eine spezifische Stelle beinhaltet, die dem ersten Ende näher ist als dem zweiten Ende, wobei die spezifische Stelle biegbar, dehnbar und in der Form veränderbar ist, und wobei sich die wiederverwendbare fluidundurchlässige Barriere von der ersten Konfiguration in die zweite Konfiguration als Reaktion auf Biegen und Dehnen der spezifischen Stelle umwandelt.

3. Fluidsammelanordnung nach Anspruch 1 oder 2, wobei die wiederverwendbare fluidundurchlässige Barriere und die mindestens eine Einweg-Dochtpatrone mindestens einen Spalt zwischen der mindestens einen Einweg-Dochtpatrone und dem ersten Ende definieren, und/oder die wiederverwendbare fluidundurchlässige Barriere und die mindestens eine Einweg-Dochtpatrone mindestens einen Spalt zwischen der mindestens einen Einweg-Dochtpatrone und dem zweiten Ende definieren.

4. Fluidsammelanordnung nach einem der vorstehenden Ansprüche, die ferner mindestens ein Rohr (108) umfasst, das mindestens teilweise in der Kammer angeordnet ist; und wobei die mindestens eine Einweg-Dochtpatrone einen Durchgang definiert, der sich mindestens teilweise dahindurch erstreckt, der dazu konfiguriert ist, das mindestens eine Rohr aufzunehmen.

5. Fluidsammelanordnung nach Anspruch 4, wobei das mindestens eine Rohr einen oder mehrere Schlitze darin gebildet beinhaltet.

6. Fluidsammelanordnung nach einem der Ansprüche 4 und 5, wobei sich der Durchgang vollständig durch die mindestens eine Einweg-Dochtpatrone erstreckt.

7. Fluidsammelanordnung nach einem der Ansprüche 4-6, wobei die mindestens eine Einweg-Dochtpatrone ferner einen Stopfen, der in dem Durchgang angeordnet ist, beinhaltet.

8. Fluidsammelanordnung nach einem der vorstehenden Ansprüche, wobei die mindestens eine Einweg-Dochtpatrone einen fluiddurchlässigen Träger, eine fluiddurchlässige Membran und eine Klebeschicht, die zwischen mindestens einem Abschnitt des fluiddurchlässigen Trägers und einem entsprechenden Abschnitt der fluiddurchlässigen Membran angeordnet ist, beinhaltet.

9. Fluidsammelanordnung nach einem der vorstehenden Ansprüche, wobei die mindestens eine Einweg-Dochtpatrone eine oder mehrere Markierungen darauf beinhaltet, wobei die eine oder die mehreren Markierungen einem oder mehreren Merkmalen der wiederverwendbaren fluidundurchlässigen Barriere entsprechen.

10. Fluidsammelanordnung nach Anspruch 9, wobei das eine oder die mehreren Merkmale der wiederverwendbaren fluidundurchlässigen den Barriere eine oder mehrere Markierungen darauf beinhalten.

11. Fluidsammelanordnung nach einem der vorstehenden Ansprüche, wobei die mindestens eine Einweg-Dochtpatrone mindestens eine Außenoberfläche beinhaltet, die eine Größe und Form darlegt, die der Größe und Form mindestens eines Abschnitts mindestens einer Innenoberfläche der wiederverwendbaren fluidundurchlässigen Barriere entsprechen.

12. Fluidsammelanordnung nach einem der vorstehenden Ansprüche, wobei die mindestens eine Einweg-Dochtpatrone mindestens teilweise aus einem Vliesmaterial gebildet ist.

13. Verfahren zum Verwenden einer Fluidsammelanordnung nach einem der Ansprüche 1-12, wobei das Verfahren Folgendes umfasst:
Bereitstellen der Fluidsammelanordnung, die eine wiederverwendbare fluidundurchlässige Barriere und eine erste Einweg-Dochtpatrone beinhaltet, wobei:
die wiederverwendbare fluidundurchlässige Barriere ein erstes Ende und ein zweites Ende, das dem ersten Ende entgegengesetzt ist, beinhaltet, die wiederverwendbare fluidundurchlässige Barriere mindestens eine Innenoberfläche beinhaltet, die eine Kammer definiert, die sich zwischen dem ersten Ende und dem zweiten Ende erstreckt, die wiederverwendbare fluidundurchlässige Barriere auch einen Auslass und mindestens eine Öffnung, die von dem Auslass beabstandet ist, definiert, die beide Zugang zu der Kammer bereitstellen, wobei die wiederverwendbare fluidundurchlässige Barriere dazu konfiguriert ist, zwischen einer ersten Konfiguration und einer zweiten Konfiguration umgewandelt zu werden;
die erste Einweg-Dochtpatrone dazu konfiguriert ist, Fluid aufzunehmen;
mit der wiederverwendbaren fluidundurchlässigen Barriere in der zweiten Konfiguration, Einsetzen der ersten Einweg-Dochtpatrone in der Kammer; und
nach dem Einsetzen der mindestens einen ersten Einweg-Dochtpatrone in die Kammer, Umwandeln der wiederverwendbaren fluidundurchlässigen Barriere von der zweiten Konfiguration in die erste Konfiguration.

14. Verfahren nach Anspruch 13, wobei das Einsetzen der ersten Einweg-Dochtpatrone in der Kammer das Positionieren mindestens eines Rohrs beinhaltet, das in der Kammer in einem Durchgang angeordnet ist, der von der ersten Einweg-Dochtpatrone definiert wird.

15. Verfahren nach einem der Ansprüche 13 und 14, das ferner nach dem Umwandeln der wiederverwendbaren fluidundurchlässigen Barriere von der zweiten Konfiguration in die erste Konfiguration Folgendes umfasst:
Umwandeln der wiederverwendbaren fluidundurchlässigen Barriere von der ersten Konfiguration in die zweite Konfiguration;
Entfernen der ersten Einweg-Dochtpatrone aus der Kammer; und ferner nach dem Entfernen der ersten Einweg-Dichtpatrone aus der Kammer Folgendes umfasst:
Einsetzen einer zweiten Einweg-Dochtpatrone in die Kammer; und
Umwandeln der wiederverwendbaren fluidundurchlässigen Barriere von der ersten Konfiguration in die zweite Konfiguration.

## Revendications

1. Ensemble de collecte de fluide (100), comprenant :
une barrière imperméable aux fluides lavable et réutilisable (102) formée à partir d'un matériau extensible et pliable incluant une première extrémité et une seconde extrémité opposée à la première extrémité, la barrière imperméable aux fluides réutilisable incluant au moins une surface intérieure définissant une chambre (104) s'étendant entre la première extrémité (110) et la seconde extrémité (112), la barrière imperméable aux fluides réutilisable définissant également une sortie (116) et au moins une ouverture (114) espacée de la sortie qui fournissent toutes les deux un accès à la chambre ; et
au moins une cartouche à effet de mèche à usage unique (106) configurée pour recevoir un fluide ;
dans lequel la barrière imperméable aux fluides réutilisable est configurée pour être transformée par pliage et étirement entre une première configuration (Fig. 2A et F) et une seconde configuration (Fig. 2B - E), dans lequel la barrière imperméable aux fluides réutilisable ne permet pas à la au moins une cartouche à effet de mèche à usage unique d'être insérée dans et/ou retirée de la chambre quand la barrière imperméable aux fluides réutilisable est dans la première configuration et la barrière imperméable aux fluides réutilisable permet à la au moins une cartouche à effet de mèche à usage unique d'être insérée dans et retirée de la chambre à travers l'ouverture quand la barrière imperméable aux fluides réutilisable est dans la seconde configuration.

2. Ensemble de collecte de fluide selon la revendication 1, dans lequel la barrière imperméable aux fluides réutilisable inclut un emplacement spécifique qui est plus proche de la première extrémité que de la seconde extrémité, l'emplacement spécifique est pliable, extensible et de forme modifiable, et dans lequel la barrière imperméable aux fluides réutilisable se transforme de la première configuration dans la seconde configuration en réponse au pliage et à l'étirement de l'emplacement spécifique.

3. Ensemble de collecte de fluide selon les revendications 1 ou 2, dans lequel la barrière imperméable aux fluides réutilisable et la au moins une cartouche à effet de mèche à usage unique définissent au moins un espace entre la au moins une cartouche à effet de mèche à usage unique et la première extrémité, et/ou la barrière imperméable aux fluides réutilisable et la au moins une cartouche à effet de mèche à usage unique définissent au moins un espace entre la au moins une cartouche à effet de mèche à usage unique et la seconde extrémité.

4. Ensemble de collecte de fluide selon l'une quelconque des revendications précédentes, comprenant en outre au moins un tube (108) disposé au moins partiellement dans la chambre ; et dans lequel la au moins une cartouche à effet de mèche à usage unique définit un passage s'étendant au moins partiellement à travers celle-ci qui est configuré pour recevoir le au moins un tube.

5. Ensemble de collecte de fluide selon la revendication 4, dans lequel le au moins un tube inclut une ou plusieurs fentes formées dans celui-ci.

6. Ensemble de collecte de fluide selon l'une ou l'autre des revendications 4 et 5, dans lequel le passage s'étend complètement à travers la au moins une cartouche à effet de mèche à usage unique.

7. Ensemble de collecte de fluide selon l'une quelconque des revendications 4-6, dans lequel la au moins une cartouche à effet de mèche à usage unique inclut en outre un bouchon disposé dans le passage.

8. Ensemble de collecte de fluide selon l'une quelconque des revendications précédentes, dans lequel la au moins une cartouche à effet de mèche à usage unique inclut un support perméable aux fluides, une membrane perméable aux fluides et une couche adhésive disposée entre au moins une partie du support perméable aux fluides et une partie correspondante de la membrane perméable aux fluides.

9. Ensemble de collecte de fluide selon l'une quelconque des revendications précédentes, dans lequel la au moins une cartouche à effet de mèche à usage unique inclut un ou plusieurs marquages sur celle-ci, les un ou plusieurs marquages correspondant à une ou plusieurs caractéristiques de la barrière imperméable aux fluides réutilisable.

10. Ensemble de collecte de fluide selon la revendication 9, dans lequel les une ou plusieurs caractéristiques de la barrière imperméable aux fluides réutilisable incluent un ou plusieurs marquages additionnels sur celle-ci.

11. Ensemble de collecte de fluide selon l'une quelconque des revendications précédentes, dans lequel la au moins une cartouche à effet de mèche à usage unique inclut au moins une surface extérieure présentant une taille et une forme qui correspondent à une taille et une forme d'au moins une partie d'au moins une surface intérieure de la barrière imperméable aux fluides réutilisable.

12. Ensemble de collecte de fluide selon l'une quelconque des revendications précédentes, dans lequel la au moins une cartouche à effet de mèche à usage unique est formée au moins partiellement à partir d'un matériau non-tissé.

13. Procédé d'utilisation de l'ensemble de collecte de fluide selon l'une quelconque des revendications 1-12, le procédé comprenant :
la fourniture de l'ensemble de collecte de fluide incluant une barrière imperméable aux fluides réutilisable et une première cartouche à effet de mèche à usage unique, dans lequel :
la barrière imperméable aux fluides réutilisable inclut une première extrémité et une seconde extrémité opposée à la première extrémité, la barrière imperméable aux fluides réutilisable incluant au moins une surface intérieure définissant une chambre s'étendant entre la première extrémité et la seconde extrémité, la barrière imperméable aux fluides réutilisable définissant également une sortie et au moins une ouverture espacée de la sortie qui fournissent toutes les deux un accès à la chambre, dans lequel la barrière imperméable aux fluides réutilisable est configurée pour être transformée entre une première configuration et une seconde configuration ;
la première cartouche à effet de mèche à usage unique est configurée pour recevoir un fluide ;
avec la barrière imperméable aux fluides réutilisable dans la seconde configuration, l'insertion de la première cartouche à effet de mèche à usage unique dans la chambre ; et
après l'insertion de l'au moins une cartouche à effet de mèche à usage unique dans la chambre, la transformation de la barrière imperméable aux fluides réutilisable de la seconde configuration dans la première configuration.

14. Procédé selon la revendication 13, dans lequel l'insertion de la première cartouche à effet de mèche à usage unique dans la chambre inclut le positionnement d'au moins un tube disposé dans la chambre dans un passage défini par la première cartouche à effet de mèche à usage unique.

15. Procédé selon l'une ou l'autre des revendications 13 et 14, comprenant en outre, après la transformation de la barrière imperméable aux fluides réutilisable de la seconde configuration dans la première configuration :
la transformation de la barrière imperméable aux fluides réutilisable de la première configuration dans la seconde configuration ;
l'enlèvement de la première cartouche à effet de mèche à usage unique de la chambre ; et comprenant en outre, après l'enlèvement de la première cartouche à effet de mèche à usage unique de la chambre :
l'insertion d'une seconde cartouche à effet de mèche à usage unique dans la chambre ; et
la transformation de la barrière imperméable aux fluides réutilisable de la seconde configuration dans la première configuration.
